# FASCICULE DE BREVET EUROPEEN

(11) **EP 0 749 498 B1**
(45) Date de publication et mention de la délivrance du brevet: **05.08.1998**
(21) Numéro de dépôt: 95911377.0
(22) Date de dépôt: 06.03.1995
(51) Int. Cl.: C12Q 1/68

(54) **MARQUEURS GENETIQUES UTILISES CONJOINTEMENT POUR LE DIAGNOSTIC DE LA MALADIE D'ALZHEIMER, METHODE ET KIT DE DIAGNOSTIC**
GEMEINSCHAFTLICH VERWENDETE GENETISCHE MARKER ZUR DIAGNOSTIK DER ALZHEIMERSCHEN KRANKHEIT, METHODE UND DIAGNOSTISCHER REAGENZIENSATZ
COMBINED USE OF GENETIC MARKERS FOR THE DIAGNOSIS OF ALZHEIMER'S DISEASE, DIAGNOSTIC KIT AND METHOD

(30) Priorité: 07.03.1994 FR 9402603
(43) Date de publication de la demande: 27.12.1996
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: AMOUYEL, Philippe, F-59700 Marcq-en-Baroeul (FR); CHARTIER-HARLIN, Marie-Christine, F-59650 Villeneuve-d'Asq (FR)
(74) Mandataire: Le Guen, Gérard
(86) Numéro de dépôt international: FR9500259
(87) Numéro de publication internationale: WO9524504

(56) Documents cités:
- WO-A-94/09155
- THE LANCET, vol. 342, no. 8882, 20 Novembre 1993 LANCET,LONDON,UK, pages 1308-1309, N. ANWAR ET AL. 'Apolipoprotein E-epsilon4 allele and Alzheimer's disease'
- ANNALS OF NEUROLOGY, vol. 31, no. 2, Février 1992 LISSLE, BROWN AND COMPANY, BOSTON, MA, US;, pages 223-227, G.D. SCHELLENBERG ET AL. 'Genetic association and linkage analysis of the apolipoprotein CII locus and familial Alzheimer's disease' cité dans la demande
- NEUROLOGY, vol. 43, no. 8, Août 1993 EDGELL COMMUN. INC., DULUTH, MINN, US;, pages 1467-1472, A.M. SAUNDERS ET AL. 'Association of apolipoprotein E allele epsilonç with late-onset familial and sporadic Alzheimer's disease' cité dans la demande
- SCIENCE, vol. 261, 13 Août 1993 AAAS, WASHINGTON, DC, US;, pages 921-923, E.H. CORDER ET AL. 'Gene dose of apolipoprotein E type 4 allele and the risk of Alzheimer disease in late onset families' cité dans la demande
- PROC. NATL. ACAD SCI., vol. 90, no. 5, 1 Mars 1993 NATL. ACAD SCI., WASHINGTON, DC, US;, pages 1977-1981, W.J. SCHRITTMACHER ET AL. 'Apolipoprotein E: High-avidity binding to beta-amyloid and increased frequency of type 4 allele in late-onset familial Alzheimer disease' cité dans la demande
- AM J. HUMAN GENETICS, vol. 44, no. 3, Mars 1989 AM. SOC. HUM. GENET., UNIV. CHICAGO PRESS, US;, pages 388-396, J.L. WEBER AND P.E. MAY 'Abundant class of human DNA polymorphisms which can be typed using the polymerase chain reaction' cité dans la demande
- CYTOGENETICS AND CELL GENETICS, vol. 58, no. 1-4, 1991 KARGER, NEW YORK, US;, pages 751-784, H.H. ROPERS AND M.A. PERICAK-VANCE 'Report of the committee on the genetic constitution of chromosome 19' cité dans la demande
- HUMAN MOLECULAR GENETICS, vol. 3, no. 4, Avril 1994 OXFORD UNIVERSITY PRESS, CAMBRIDGE, UK;, pages 569-574, M.-C. CHARTIER-HARLIN ET AL. 'Apolipoprotein E, epsilon4 allele as a major risk factor for sporadic early and late onset forms of Alzheimer's disease: analysis of the 19q13.2 chromosomal region'

## Description

La présente invention concerne des marqueurs génétiques utilisés conjointement pour le diagnostic de la maladie d'Alzheimer.

La maladie d'Alzheimer est une pathologie encéphalique caractérisée par une démence précoce avec une perte de neurones corticaux associée à des plaques de β-amyloïde, des enchevêtrements de neurofibrilles et dans la plupart des cas une angiopathie amyloïde. Il existe de fortes présomptions pour une influence génétique dans l'étiologie de la maladie d'Alzheimer (WO 94/01772).

Cette composante génétique a été mis en évidence depuis de nombreuses années par des observations indirectes qui suggèrent une hérédité sur le mode autosomique dominant et une pénétrance dépendante de l'age pour expliquer les liens familiaux entre des individus atteints par la maladie. Des études de génétique moléculaire récentes ont permis d'isoler des gènes putatifs de la maladie d'Alzheimer par la recherche de marqueurs génétiques polymorphes spécifiques de chromosomes (Bird et al., 1989, Neurobiology of Aging 10, 432-434).

Trois localisations chromosomiques ont été décrites comme étant impliquées dans les formes familiales à survenue précoce (âge de survenue inférieur à 60 ans) : le chromosome 21, le chromosome 14 et le chromosome 19. Deux études de liaison ont suggéré que la région chromosomique 19q13.2 était associée à des formes de maladie d'Alzheimer familiales à survenue tardive (Pericak-Vance et al, Am. J. Hum. Genet. (1991), 48, 1034-1050 ; Schellenberg et al., Ann. Neurol. (1992), 31, 223-227). Au sein de cette région chromosomique, le groupe de gènes d'apolipoprotéines (APO) E-CI-CI'-CII est une zone candidate. Parmi les produits de ces gènes, l'apolipoprotéine E (APOE) est particulièrement impliquée dans le système nerveux : APOE est présente dans les plaques séniles et possède une affinité de liaison avec le peptide β-A4. Strittmatter et al. (Proc. Natl. Acad. Sci. (1993) 90, 177-181) ont décrit une fréquence augmentée de l'allèle ε4 du gène APOE dans les formes familiales de la maladie d'Alzheimer à survenue tardive. Cette observation a été confirmée pour les formes familiales (Corder et al., Science (1993), 261, 921-923) et les formes sporadiques de la maladie d'Alzheimer (Corder et al., Science (1993), 261, 921-923 ; Saunders et al., Neurology -(1993), 13, 1467-1472).

D'autre part, Schellenberg et al. (Ann. Neurol. 1992, 31:223-227) ont rapporté une association génétique entre l'allèle F du gène de l'apolipoprotéine CII (allèle du polymorphisme de longueur de fractions de restriction de TaqI (RFLP : Restriction Fragment Length Polymorphism)) et la forme familiale de la maladie d'Alzheimer.

Les auteurs de la présente invention ont réalisé une étude portant sur deux populations d'origine différentes atteintes l'une d'une forme à survenue tardive de la maladie d'Alzheimer (après 65 ans), l'autre d'une forme à survenue précoce de la maladie d'Alzheimer (avant 65 ans), qui a permis d'établir une augmentation significative dans les deux groupes d'au moins deux des marqueurs génétiques suivants : allèle APOE ε4, allèle D19S178 court et allèle APO CII long.

La localisation sur le chromosome 19 des marqueurs APOE, APO CII (APO C2) et D19S178 est connue et décrite notamment par Williamson et al. (Cyto Genetic and Cell Genetic 1991, vol. 58, p. 1678).

La présente invention a ainsi pour objet l'utilisation conjointe d'au moins deux marqueurs génétiques choisis parmi APOE, D19S178 et APO CII pour le diagnostic de la maladie d'Alzheimer, les marqueurs génétiques étant de préférence constitués par APOE et D19S178 et/ou APO CII, de préférence encore par APOE, D19S178 et APO CII.

Les marqueurs génétiques utilisés sont avantageusement l'allèle APOE ε4, l'allèle D19S178 court et l'allèle APO CII long.

Le gène APOE possède trois allèles : ε2, ε3 et ε4.

Par allèle APO CII long, on entend un allèle comprenant plus de 30±3, de préférence plus de 30 répétitions consécutives des bases cytosine-adénine.

Par allèle D19S178 court, on entend un allèle comprenant moins de 167±4, de préférence moins de 167 nucléotides.

L'invention a également pour objet un procédé de diagnostic de la maladie d'Alzheimer caractérisé en ce que l'on recherche dans un échantillon biologique d'un patient la présence de deux au moins des marqueurs suivants : allèle APOE ε4, allèle D19S178 court et allèle APO CII long.

La méthode selon l'invention comprend avantageusement les étapes suivantes :
a) mise en contact de l'échantillon biologique contenant de l'ADN avec un couple d'amorces spécifiques permettant l'amplification de tout ou d'une partie des gènes APOE, D19S178 et/ou APO CII, l'ADN humain contenu dans l'échantillon ayant été éventuellement rendu accessible à l'hybridation et dans des conditions permettant une hybridation des amorces à l'ADN humain contenu dans l'échantillon biologique ;
b) amplification de l'ADN humain ;
c) révélation des produits d'amplification par les techniques appropriées ;
d) détection de la présence éventuelle des allèles APOE ε4, D19S178 court et APO CII long par les techniques appropriées.

Par diagnostic, au sens de la présente invention, on entend la confirmation de la présence d'au moins deux marqueurs choisis parmi ceux décrits ci-dessus chez des patients dont le tableau clinique fait état d'une symptomatologie pouvant être attribuée à la maladie d'Alzheimer, ou encore une probabilité accrue chez des sujets de développer la maladie d'Alzheimer par rapport à l'ensemble d'une population, l'accroissement de la probabilité étant d'au moins un facteur 4.

L'échantillon biologique peut être le sang total, la fraction leucocytaire du sang ou encore un tissu à partir duquel de l'ADN peut être extrait, ou un fluide biologique.

Les amorces spécifiques utilisées dans le cadre de la présente invention peuvent être notamment celles décrites dans Roppers et al. (1991), Cytogenet. Cell Genet. 58, 751-784) pour D19S178, Hixson et al. (1990), J. Lipid. Res. 31, 545-548 pour APOE et Weber et al., Am. J. Hum. Genet. 44, 388-396 pour APO CII.

D'autres amorces peuvent convenir, le critère de sélection des amorces étant qu'elles permettent l'amplification des parties des gènes APOE, D19S178 et APO CII comportant les polymorphismes respectifs : région 112-158 de APOE, extrémité 5' de APO CII.

Les allèles recherchés, notamment D19S178 et APO CII long, peuvent être mis en évidence par détermination de la longueur des fragments amplifiés, par exemple par une électrophorèse sur gel de polyacrylamide ou par détermination de la séquence du fragment amplifié.

Les allèles recherchés, notamment APO ε4, peuvent également être détectés par la technique d'analyse de polymorphisme conformationnel de simples brins ("Single-Strand Conformation Polymorphism" (SSCP) telle que décrite par Masato Orita et al., ou encore par hybridation de sonde à l'aide d'une sonde spécifique.

La présente invention a également pour objet un nécessaire pour la mise en oeuvre du procédé selon l'invention, dans un échantillon comprenant les éléments suivants :
- des couples d'amorces spécifiques des gènes APOE, D19S178 et APO CII,
- les réactifs nécessaires pour effectuer une amplification d'ADN,
- éventuellement les réactifs permettant la détection des allèles APOE ε4, D19S178 court et/ou APO CII long, par exemple des réactifs nécessaires à une électrophorèse sur gel de polyacrylamide et la révélation des fragments après migration
- éventuellement des standards de référence constitués par les allèles sauvages des gènes mutants.

La présente invention a également pour objet des produits d'amplification de tout ou partie d'au moins deux gènes choisis parmi APOE, APO CII et D19S178.

Elle vise également une composition de diagnostic constituée d'au moins deux marqueurs génétiques choisis parmi APOE, APO CII et D19S178.

On rapportera ci-après les résultats de l'étude des inventeurs auteurs de la présente invention, montrant l'implication des marqueurs APOE, APO CII et D19S178 dans la fréquence de survenue de la maladie d'Alzheimer.

L'étude a porté sur deux groupes de patients atteints de la maladie d'Alzheimer, un groupe français composé de 36 patients atteints d'une forme à survenue tardive de la maladie d'Alzheimer (78±9 ans ; intervalle 65-91 ans) et un groupe britannique composé de 34 patients présentant une forme à survenue précoce de la maladie d'Alzheimer (57±4 ans ; intervalle : 49-64 ans) diagnostiquées par la clinique.

Ces deux groupes ont été comparés à deux groupes témoins de mêmes origine et âges, composés respectivement de 38 et 36 sujets dont l'environnement était identique à celui des patients atteints de la maladie d'Alzheimer.

L'ADN génomique des patient a été extrait des leucocytes selon la méthode décrite par Marcadet et al. (Standardized Southern-Blot Workshop Technique-Histocompatibility Testing, Springer Verlag, New-York, Vol. 1). L'ADN génomique a été amplifié par PCR à l'aide d'un appareil d'amplification Perkin Elmer Cetus.

Cinq marqueurs localisés dans la région chromosomique 19q13.2 ont été étudiés comme décrit dans les références correspondantes : deux marqueurs anonymes contenant des motifs (CA)ₙ , D19S47 (référencé sous Mfd 9 (Weber et al. (1989), Am. J. Hum. Genet. 44, 388-396)) et D19S178 (référencé sous Mfd 139 (Ropers et al. (1991) Cytogenet. Cell Genet. 58, 751-784)), le polymorphisme APOE (Hixson et al. (1990) J. Lipid. Res. 31, 545-548), le polymorphisme de longueur de fragments de restriction (RFLP) pour HpaI de l'extrémité 5' du locus du gène Apo CI (Nillesen et al. (1990), Nucleic Acids Res. 18, 3428) et le polymorphisme de motifs répétitifs (CA)ₙ dans le gène APO CII (référencé sous Mfd 5) (Weber et al., Am. J. Hum. Genet. 44, 388-396).

Les analyses statistiques ont été réalisées avec un logiciel SAS version 6,04. Des analyses "Univariates" ont été réalisées à l'aide du test χ² de Pearson et du test exact de Fisher si nécessaire. Compte tenu de la rareté de la fréquence de certains allèles observée pour des polymorphismes microsatellites, les allèles ont été regroupés en deux groupes en fonction de leur nombre de nucléotides. Ainsi, D19S178 a été partagé en allèles longs (167 nucléotides et au-delà) et allèles courts (moins de 167 nucléotides), les allèles de l'extrémité 5' de APO CII à motifs répétitifs ont été divisés en allèles longs (30 motifs répétitifs et au-delà) et allèles courts (moins de 30 motifs répétitifs).

Les fréquences alléliques ont été calculées par comptage des allèles.

Les résultats recueillis ont été traités par informatique en utilisant un modèle de régression logistique par étape à l'aide de tests statistiques de vraisemblance comme décrit par Breslow N.E. et al., Statistical Methods in Cancer Research, Vol. 1, pp. 192-247. L'analyse des déséquilibres de liaison a été faite comme décrit par Thompson E.A. et al., Am. J. Hum. Genet., 42, 113-124 et Hill, W.G., 1974, Hereditary, 33, 229-239.

Les fréquences des marqueurs D19S178, APOE, APO CI et APO CII ont été estimées à l'aide de l'algorythme décrit par McLean et Morton, Genet. Epidemiol. 2, 263-272, mis sous forme de programme informatique, comme décrit par Cox et al., Am. J. Hum. Genet., 43, 495-501.

Les fréquences estimées ont été comparées à celles attendues sur la base de l'équilibre du χ² de Pearson ou du test exact de Fischer.

Les résultats sont rapportés aux Tableaux I à IV ci-après.

Le tableau I rapporte les distributions de fréquence allélique des polymorphismes génomiques pour des sujets sporadiques atteints de la maladie d'Alzheimer par rapport à des témoins.

Aucune différence significative n'apparaît entre les sujets et les témoins dans la distribution de l'allèle D19S47 pour les différentes populations. Dans la population à survenue tardive de la maladie, les fréquences des polymorphismes des gênes APOE et APO CII étaient significativement différentes entre les sujets et les témoins : l'allèle APOE ε4 et les allèles APO CII longs étaient observés plus fréquemment chez les sujets à survenue tardive de la maladie d'Alzheimer que chez les témoins.

Les mêmes analyses ont été réalisées pour une population indépendante du Royaume-Uni de sujets sporadiques à survenue précoce par rapport à des témoins. Cette population a été étudiée pour les deux limites d'âge de survenue généralement acceptées, c'est-à-dire moins de 65 ans et moins de 60 ans. Pour le groupe de sujets dont l'âge de survenue était inférieur à 65 ans, les distributions alléliques des polymorphismes des gènes D19S178, APOE et APOCI étaient significativement différentes entre les sujets et les témoins : les allèles D19S178 courts, l'allèle APOE ε4 et la présence du site de restriction de HpaI dans APO CI (allèle 2) ont été plus fréquemment observés chez les malades où la survenue de la maladie était précoce que chez les témoins. Pour les groupes de sujets avec un âge de survenue de la maladie inférieure à 60 ans, les distributions alléliques des polymorphismes des gènes D19S178, APOE et APO CI étaient similaires à celles du groupe de sujets avec un age de survenue de la maladie inférieur à 65 ans.

Pour les analyses suivantes, la limite de séparation en fonction de l'âge entre le groupe à survenue précoce et le groupe à survenue tardive était de 65 ans.

La fréquence observée pour l'allèle APOE ε4 dans le groupe de malades à survenue précoce n'était pas significativement différente de celle observée dans le groupe de malades à survenue tardive, et dans les deux populations, l'allèle APOE ε2 était rare chez les sujets atteints de la maladie d'Alzheimer.

Une corrélation inverse entre l'âge de survenue et le nombre de copies de l'allèle APOE ε4 était observée dans la population à survenue tardive (p < 0,026), mais non dans la population à survenue précoce. Les âges moyens de survenue des sujets ayant deux allèles APO ε4, un allèle ε4 ou sans l'allèle APO ε4 étaient respectivement de 70,5, 75,0 et 81,2 ans dans le groupe à survenue tardive, et 58,5, 56,6 et 57,3 ans dans le groupe à survenue précoce.

Les résultats d'études de déséquilibre de liaison deux à deux entre les polymorphismes D19S178, APOE, APO CI et APO CII sont rapportés au Tableau II.

Aucune déviation de l'équilibre de Hardy-Weinberg n'était observée. Un déséquilibre de liaison complet était retrouvé entre l'allèle APO ε4 et l'allèle 2 du polymorphisme de longueur des fragments de restriction (RFLP) de APO CI dans les deux populations.

Dans la population témoin, un déséquilibre de liaison non significatif était retrouvé entre les polymorphismes D19S178 et APOE et à l'intérieur de l'ensemble APOE-CI-CI'-CII.

Les fréquences haplotypiques des 4 marqueurs (D19S178, APOE, APO CI et APO CII) ont été estimées comme rapporté au Tableau III ci-après.

Parmi les 24 haplotypes théoriques, seuls 15 étaient retrouvés avec l'algorythme par la méthode décrite par McLean et Morton. Certains haplotypes étaient observés seulement chez les malades (n° 1, 2 et 7) et d'autres seulement chez les témoins (n° 12 et 13) à la fois dans la population à survenue tardive et celle à survenue précoce. L'haplotype n° 10 était deux fois plus fréquent dans la population de malades à survenue tardive que dans la population à survenue précoce.

Le contenu d'informations du polymorphisme (Polymorphism Information Content (PIC)) et le degré d'hétérozygotie obtenus en combinant ces 4 marqueurs étaient élevés dans les deux groupes.

Les différences dans la distribution des fréquences d'haplotypes estimées entre les malades et les témoins ont été testées à l'aide du test de rapport de probabilité (likelihood ratio test) avec un nombre de degré de liberté égal à 15. Les résultats étaient de 88,4 et 93,2 respectivement pour le groupe de malades à survenue tardive et le groupe de malades à survenue précoce.

Pour estimer la tendance à développer la maladie ("odds ratio") pour les porteurs d'au moins un des allèles des différents génotypes, on a utilisé des modèles de régression logistique multiple par étapes, qui ont été adaptés aux observations.

Dans le groupe à survenue tardive, les "odds ratios" estimés étaient de 6,49 pour l'allèle APOE ε4 (intervalle de confiance à 95% = [1,68 ; 25,03]), 0,10 pour l'allèle APOE 2 (intervalle de confiance à 95% = [1,19 ; 10,70]).

Dans le groupe à survenue précoce, les "odds ratios" estimés obtenus étaient de 3,80 pour l'allèle APO ε4 (intervalle de confiance à 95% = [0,01 ; 0,85]) et 4,44 pour les allèles courts de D19S178 (intervalle de confiance à 95% = [1,27 ; 15,49]).

L'odds ratio (approximation du risque) ajusté pour les porteurs d'au moins un allèle APOE ε4 de développer une maladie d'Alzheimer sporadique de type à survenue précoce ou à survenue tardive était de 4.10 (intervalle de confiance à 95% = [1.84 ; 9.16]), comme estimé dans un modèle de régression, logistique adapté pour les données se rapportant à la population totale.

Pour les porteurs d'au moins un allèle, APOE ε2, l'odds ratio était de 0.11 (intervalle de confiance à 95% = [0,02 ; 0,50]), suggérant ainsi un effet protecteur de cet allèle.

Le risque de développer une maladie d'Alzheimer pour les porteurs d'au moins un allèle ε4 et d'au moins un des deux marqueurs : allèle D19S178 court et allèle APO CII long est rapporté au tableau IV ci-dessous.

Dans les deux populations, les risques "odds ratios" étaient significativement augmentés lorsque l'un de ces deux marqueurs était considéré en même temps que l'allèle APOE ε4.

Par exemple, le risque de déclarer une maladie d'Alzheimer à survenue tardive pour un sujet porteur d'au moins un allèle APOE ε4 et d'au moins un allèle APO CII long est maximal, cette configuration n'étant jamais retrouvée chez les témoins. Dans cette même population à début tardif, pour les porteurs d'au moins un allèle APOE ε4 et d'au moins un allèle D19S178 court, l'odds ratio s'élève à 14 (14.23). Cet odds est également augmenté (supérieur à 8 (8.68)) pour ces mêmes allèles dans le cas d'une maladie d'Alzheimer à début précoce.

Dans l'ensemble de la population, le risque de développer une maladie d'Alzheimer indépendemment de l'âge de survenue est augmenté pour les porteurs d'au moins un allèle APOE ε4 et d'au moins un allèle D19S178 court comme en témoigne l'élévation de l'odds ratio supérieur à 12 (12.46) ainsi que pour les porteurs d'au moins un APOE ε4 et d'au moins un allèle APO CII long (odds ratio supérieur à 9 (9,72)).

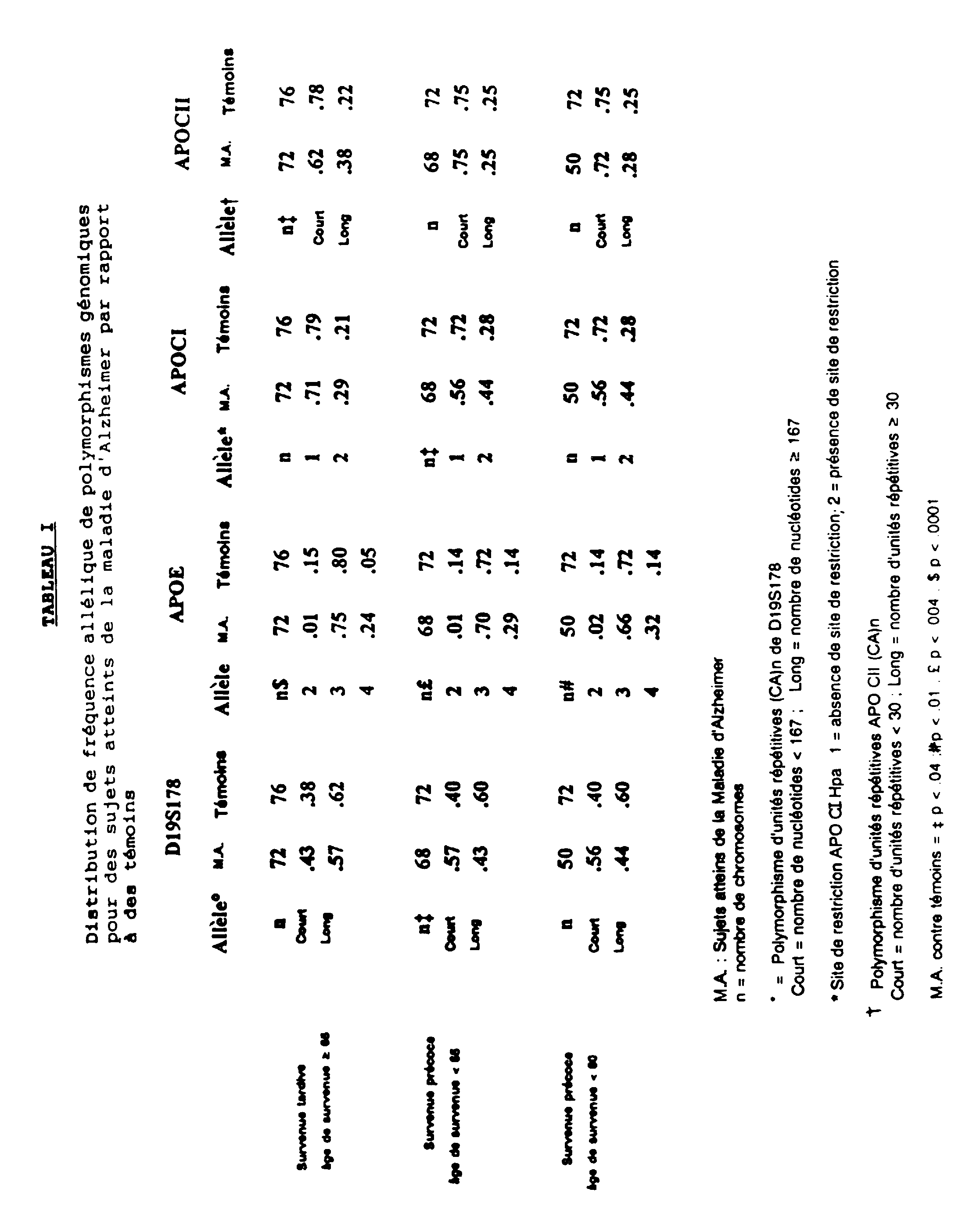

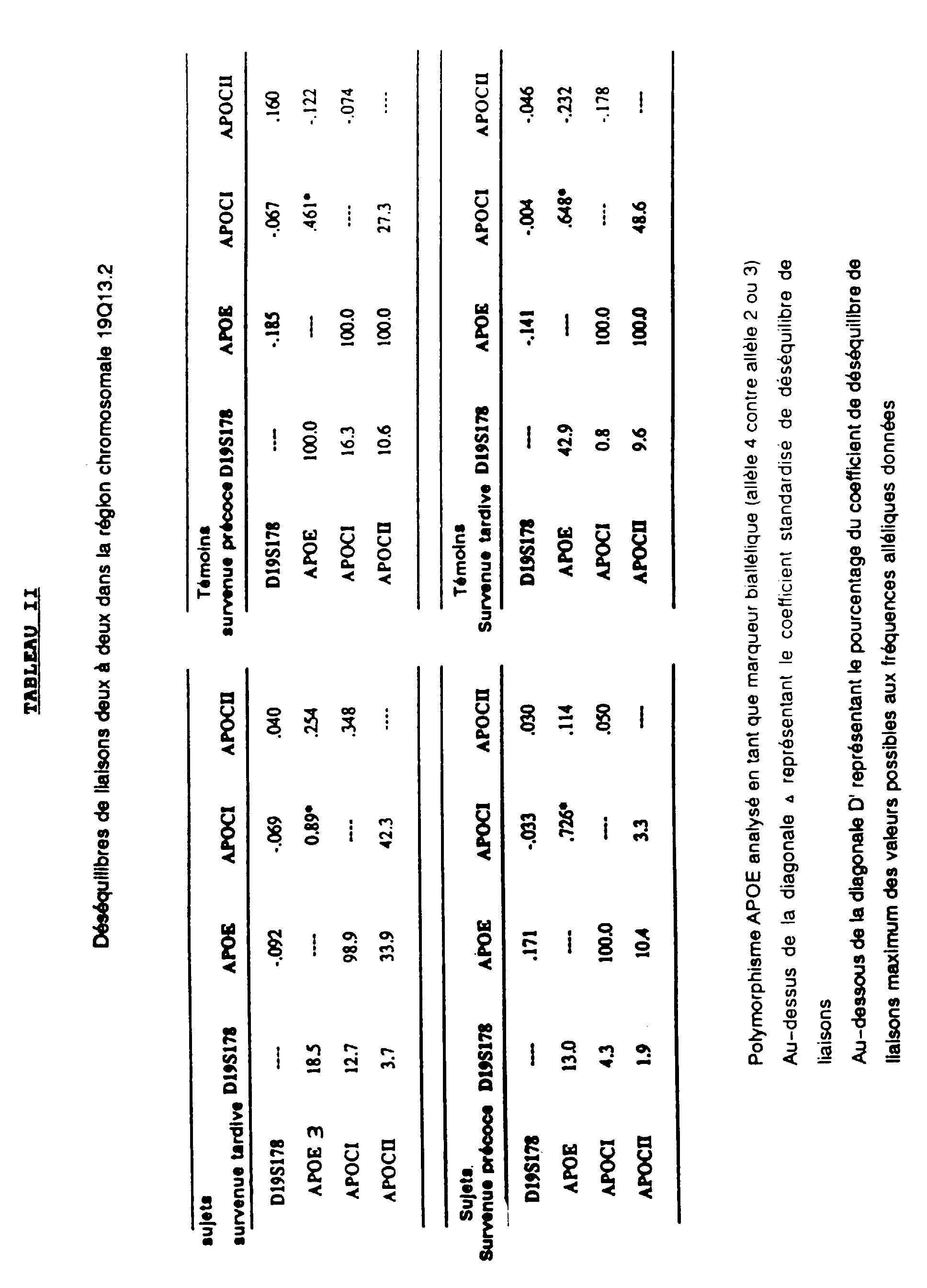

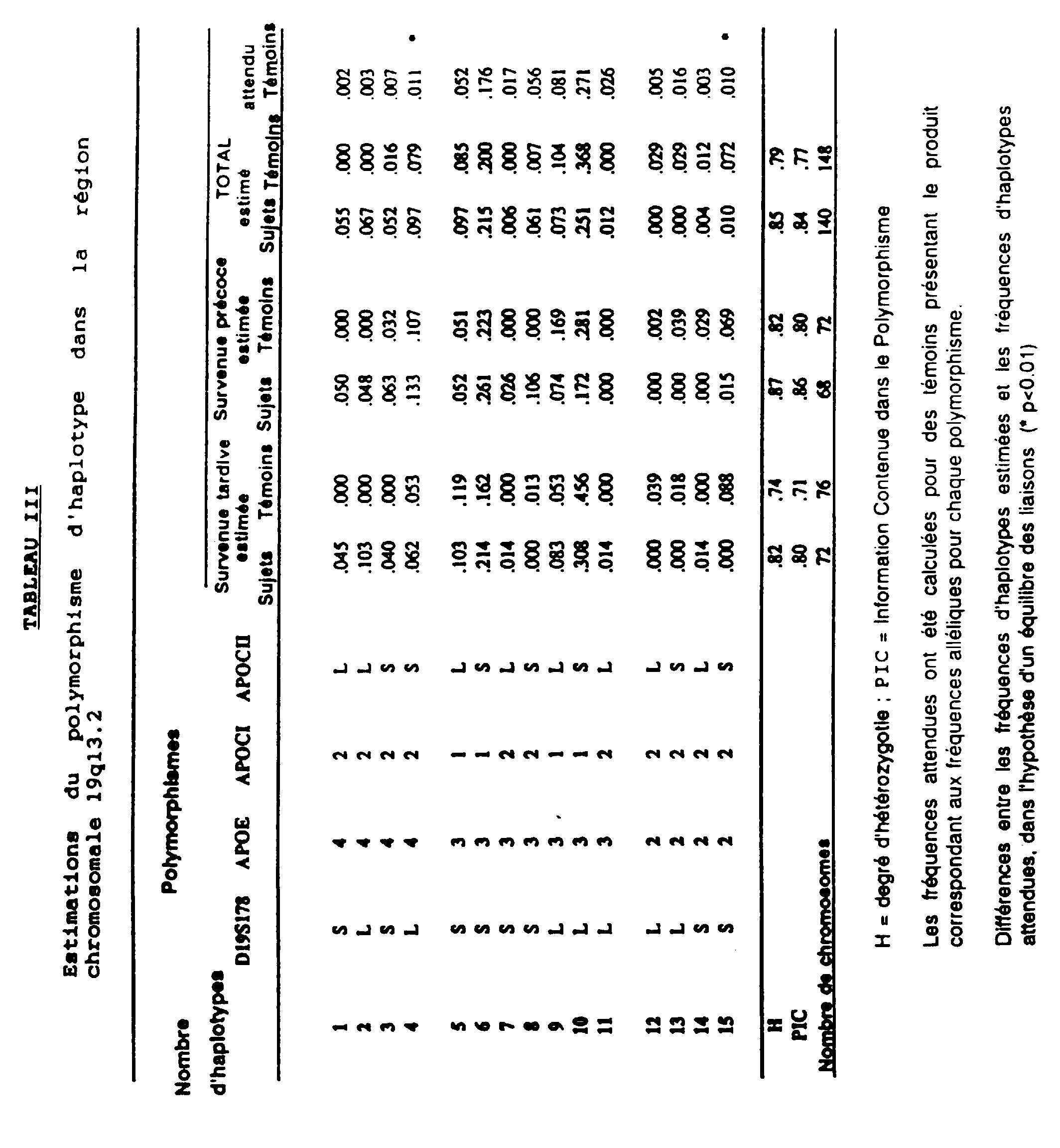

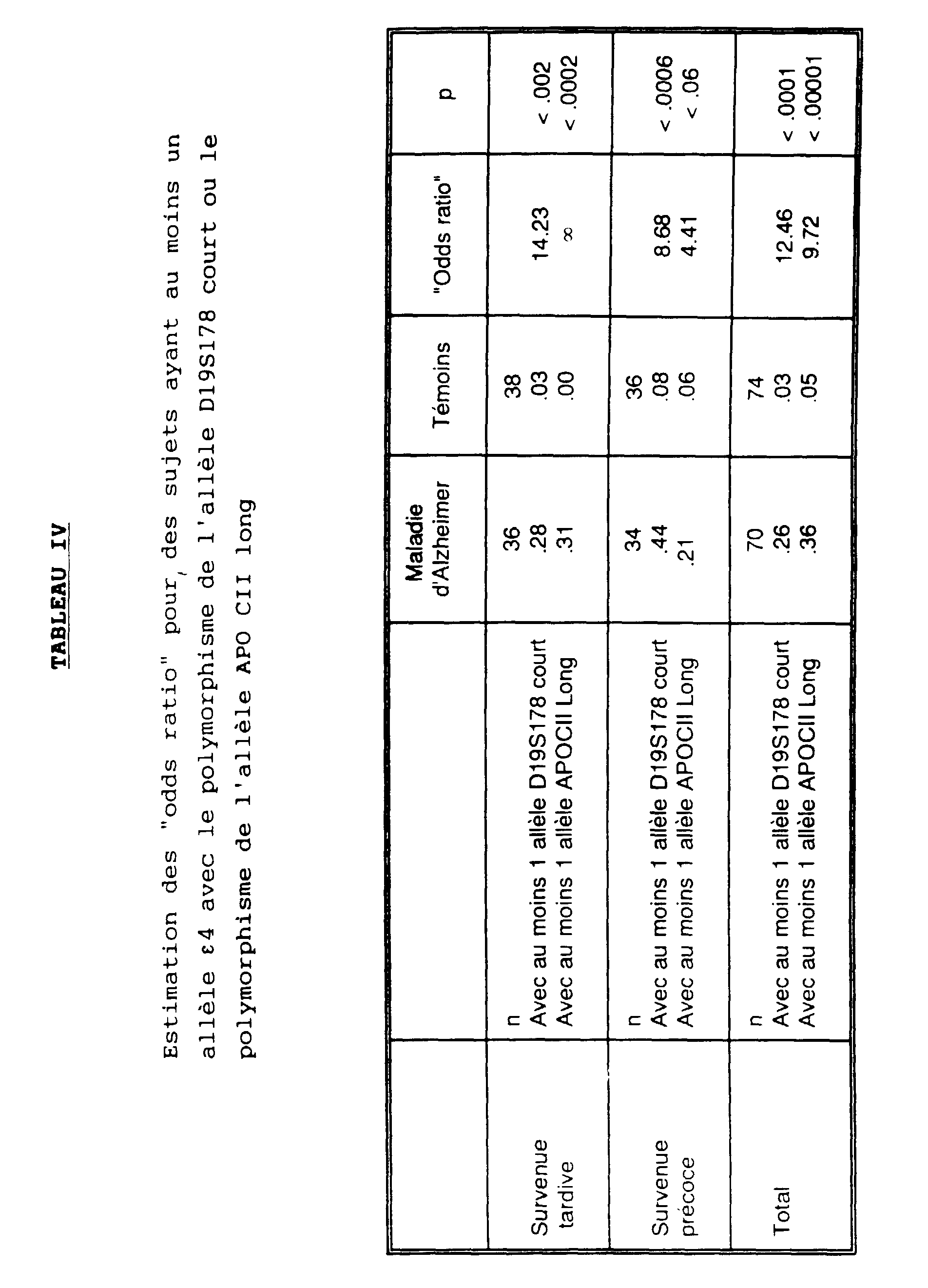

## Revendications

1. Utilisation conjointe d'au moins deux marqueurs génétiques choisis parmi APOE, D19S178 et APO CII pour le diagnostic de la maladie d'Alzheimer.

2. Utilisation selon la revendication 1 des marqueurs APOE, APO CII et/ou D19S178.

3. Utilisation selon la revendication 1 ou la revendication 2 des marqueurs APOE, D19S178 et APO CII.

4. Utilisation selon l'une quelconque des revendications précédentes, caractérisée en ce que les marqueurs sont l'allèle APOE ε4, l'allèle D19S178 court et l'allèle APO CII long.

5. Utilisation selon la revendication 4, caractérisée en ce que l'allèle D19S178 court comporte moins de 167±4, notamment moins de 167 nucléotides.

6. Utilisation selon la revendication 4, caractérisée en ce que l'allèle APO CII long comporte plus de 30±3, notamment plus de 30 motifs répétitifs (CA).

7. Méthode de diagnostic de la maladie d'Alzheimer, caractérisé en ce que l'on recherche dans un échantillon biologique d'un patient la présence de deux au moins des marqueurs suivants : allèle APOE ε4, allèle D19S178 court, et allèle APO CII long.

8. Méthode selon la revendication 7, caractérisée par les étapes suivantes :
a) mise en contact de l'échantillon biologique contenant de l'ADN avec un couple d'amorces spécifiques permettant l'amplification de tout ou partie des gènes APOE, D19S178 et/ou APO CII, l'ADN humain contenu dans l'échantillon ayant été éventuellement rendu accessible à l'hybridation et dans des conditions permettant une hybridation des amorces à l'ADN humain contenu dans l'échantillon biologique ;
b) amplification de l'ADN humain ;
c) révélation des produits d'amplification par les techniques appropriées ;
d) détection de la présence éventuelle des allèles APOE ε4, D19S178 court et APO CII long par les techniques appropriées.

9. Méthode selon la revendication 8, caractérisée en ce que la présence des allèles APOE ε4, D19S178 court et APO CII long est mise en évidence par la technique d'électrophorèse sur gel de polyacrylamide.

10. Nécessaire pour la mise en oeuvre d'une méthode selon l'une des revendications 8 à 10, caractérisé en ce qu'il comprend :
- des couples d'amorces spécifiques de deux au moins des gènes APOE, D19S178 et APO CII,
- les réactifs nécessaires pour effectuer une amplification d'ADN,
- éventuellement les réactifs permettant la détection des allèles APOE ε4, D19S178 court et/ou APO CII, par exemple des réactifs nécessaires à une électrophorèse sur gel de polyacrylamide et la révélation des fragments après migration,
- éventuellement des standards de référence constitués par les allèles sauvages des gènes mutants.

11. Produits d'amplification de tout ou partie d'au moins deux gènes choisis parmi APOE, APO CII et D19S178.

12. Composition de diagnostic constituée d'au moins deux marqueurs génétiques choisis parmi APOE, APO CII et D19S178.

## Patentansprüche

1. Gemeinsame Verwendung wenigstens zweier genetischer Marker ausgewählt aus APOE, D19S178 und APO CII zur Diagnose der Alzheimer Krankheit.

2. Verwendung nach Anspruch 1 der Marker APOE, APO CII und/oder D19S178.

3. Verwendung nach Anspruch 1 oder 2 der Marker APOE, D19S178 und APO CII.

4. Verwendung nach einem der vorhergehenden Ansprüche, dadurch gekennzeichnet, daß die Marker das Allel APOE E4, das Allel D19S178 kurz und das Allel APO CII lang sind.

5. Verwendung nach Anspruch 4, dadurch gekennzeichnet, daß das Allel D19S178 kurz weniger als 167±4, insbesondere weniger als 167 Nukleotide umfaßt.

6. Verwendung nach Anspruch 4, dadurch gekennzeichnet, daß das Allel APO CII lang mehr als 30±3, insbesondere mehr als 30 repetetive Motive (CA) umfaßt.

7. Verfahren zur Diagnose der Alzheimer Krankheit, dadurch gekennzeichnet, daß man in einer biologischen Probe eines Patienten wenigstens zwei der folgenden Marker sucht: Allel APOE E4, Allel D19S178 kurz und Allel APO CII lang.

8. Verfahren nach Anspruch 7, gekennzeichnet durch die folgenden Stufen:
a) In Kontakt bringen einer DNA enthaltenden biologischen Probe mit einem Paar spezifischer Sonden, so daß die Amplifizierung eines Teils der oder der ganzen Gene APOE, D19S178 und/oder APO CII ermöglicht wird, wobei die menschliche DNA, die in der Probe enthalten ist, gegebenenfalls in einen der Hybridisierung zugänglichen Zustand und unter Bedingungen gebracht wurde, die eine Hybridisierung der Sonden mit der menschlichen DNA, welche in der biologischen Probe enthalten ist, ermöglichen;
b) Verstärkung der menschlichen DNA;
c) Auffinden der amplifizierten Produkte durch geeignete Techniken;
d) Nachweis von gegebenenfalls vorhandenen Allelen APOE E4, D19S178 kurz und APO CII lang durch geeignete Techniken.

9. Verfahren nach Anspruch 8, dadurch gekennzeichnet, daß das Vorhandensein der Allele APOE E4, D10S178 kurz und APO CII lang durch eine Polyacrylamidgelelektrophorese nachgewiesen wird.

10. Test-Kit zum Durchführen eines Verfahrens nach den Ansprüchen 8 bis 9, dadurch gekennzeichnet, daß er enthält:
- Paare von spezifischen Sonden von mindestens zweien der Gene APOE, D19S178 und APO CII,
- gegebenenfalls Reagentien, die den Nachweis der Allele APOE E4, D19S178 kurz und/oder APO CII erlauben, beispielsweise die zur Durchführung einer Polyacrylamidgelelektrophorese und zum Nachweis von Fragmenten nach der Migration erforderlichen Reagentien,
- gegebenenfalls Standards zum Vergleich der Wildallele der Genmutanten.

11. Verstärkungsprodukt der gesamten oder eines Teils von mindestens zwei Genen, ausgewählt aus APOE, APO CII und D19S178.

12. Diagnostische Zubereitung gebildet aus mindestens zwei Genmarkern ausgewählt aus APOE, APO CII und D19S178.

## Claims

1. Combined use of at least two genetic markers selected from APOE, D19S178 and APO CII for the diagnosis of Alzheimer's disease.

2. Use according to Claim 1 of the markers APOE, APO CII and/or D19S178.

3. Use according to Claim 1 of Claim 2 of the markers APOE, D19S178 and APO CII.

4. Use according to any one of the preceding claims, characterised in that the markers are the allele APOE E4, the short allele D19S178 and the long allele APO CII.

5. Use according to Claim 4, characterised in that the short allele D19S178 comprises less than 167±4, in particular less than 167 nucleotides.

6. Use according to Claim 4, characterised in that the long allele APO CII comprises more than 30±3, in particular more than 30 repetitive patterns (CA).

7. Method of diagnosis for Alzheimer's disease, characterised in that the presence of at least two of the following markers is sought in a biological sample of a patient: allele APOE E4, the short allele D19S178 and the long allele APO CII.

8. Method according to Claim 7, characterised by the following steps:
a) placing the biological sample containing DNA in contact with a pair of specific activators allowing the amplification of all or part of the genes APOE, D19S178 and/or APO CII, the human DNA contained in the sample having possibly been made accessible to hybridisation and in conditions allowing hybridisation of the activators to the human DNA contained in the biological sample;
b) amplification of the human DNA;
c) disclosure of products of amplification by appropriate techniques;
d) detection of the possible presence of the alleles APOE ε4, short D19S178 and long APO CII by appropriate techniques.

9. Method according to Claim 8, characterised in that the presence of the alleles APOE ε4, short D19S178 and long APO CII is proven by the technique of electrophoresis on polyacrylamide gel.

10. Kit for conducting a method according to one of Claims 8 to 10, characterised in that it comprises:
- pairs of specific activators of at least two of the genes APOE, D19S178 and APO CII,
- the necessary reagents for conducting the amplification of DNA,
- possibly reagents allowing the detection of the alleles APOE ε4, short D19S178 and/or APO CII, for example necessary reagents for electrophoresis on polyacrylamide gel and the disclosure of fragments after migration,
- possibly standards of reference formed by wild alleles of mutant genes.

11. Products of amplification of all or part of at least two genes selected from APOE, APO CII and D19S178.

12. Diagnostic composition formed from at least two genetic markers selected from APOE, APO CII and D19S178.
